# FASCICULE DE BREVET EUROPEEN

(11) **EP 1 844 232 B1**
(45) Date de publication et mention de la délivrance du brevet: **10.08.2011**
(21) Numéro de dépôt: 06709473.0
(22) Date de dépôt: 02.02.2006
(51) Int. Cl.: F03G 7/00

(54) **ACTIVATION CHIMIQUE D'UN ACTIONNEUR OU D'UN MOTEUR OSMOTIQUE**
CHEMISCHE AKTIVIERUNG EINES OSMOTISCHEN STELLGLIEDS ODER EINES OSMOTISCHEN MOTORS
CHEMICAL ACTIVATION OF AN OSMOTIC ACTUATOR OR AN OSMOTIC MOTOR

(30) Priorité: 03.02.2005 FR 0550314
(43) Date de publication de la demande: 17.10.2007
(73) Titulaire: UNIVERSITE JOSEPH FOURIER, 38041 Grenoble Cédex 9 (FR); INSTITUT NATIONAL POLYTECHNIQUE DE GRENOBLE, 38031 Grenoble Cédex 1 (FR)
(72) Inventeur: LENOUVEL, François, F-38100 Grenoble (FR); DURRIEU, Vanessa, F-07200 Saint Etienne De Fontbellon (FR); BELGACEM, Naceur, F-38320 Brie Et Angonnes (FR); CINQUIN, Philippe, F-38330 Saint Nazaire Les Eymes (FR)
(74) Mandataire: de Beaumont, Michel
(86) Numéro de dépôt international: PCT/FR2006/050092
(87) Numéro de publication internationale: WO 2006/082345

(56) Documents cités:
- EP-A- 1 481 165
- US-A- 3 894 538
- US-A- 4 522 698

## Description

La présente invention concerne des dispositifs pouvant servir d'actionneurs ou de moteurs qui sont simples à réaliser, qui utilisent un carburant à faible coût, et qui émettent peu ou pas de déchets.

En outre, la présente invention concerne des dispositifs pouvant servir d'actionneurs ou de moteurs adaptés à fonctionner à l'intérieur d'un milieu biologique tel que le corps humain ou un corps animal.

De tels actionneurs et de tels moteurs trouvent des applications dans le domaine médical par exemple pour pallier la déficience d'un muscle naturel. Les muscles pouvant être remplacés ou assistés, de façon temporaire ou définitive, sont, par exemple, le muscle cardiaque, les muscles respiratoires, les sphincters et les muscles lisses ou striés, en particulier squelettiques.

De tels actionneurs et de tels moteurs trouvent également des applications dans des domaines autres que médicaux. En particulier, un tel moteur peut être utilisé dans tous les domaines dans lesquels une faible production de déchets est un facteur important dans le choix du moteur. Il peut s'agir, par exemple, du domaine automobile où l'on cherche à réduire le plus possible les déchets polluants produits par le moteur utilisé pour l'entraînement des roues du véhicule.

La demande de brevet US 2004/248269, au nom de la demanderesse, décrit un actionneur osmotique destiné à être plongé dans un milieu biologique et comprenant une enceinte déformable ayant une membrane semi-perméable, cette enceinte contenant un soluté susceptible d'être osmotiquement actif.

La demande de brevet EP-A-1481165, au nom de la demanderesse, décrit un actionneur et un moteur osmotique dont le fonctionnement peut être contrôlé avec davantage de précision. Pour ce faire, la demande de brevet EP-A-1481165 prévoit l'utilisation de microorganismes qui sont contenus dans une enceinte perméable à un solvant et non perméable à un premier soluté. Les microorganismes sont adaptés à transformer un second soluté en le premier soluté. Une chambre déformable est reliée à l'enceinte et peut augmenter de volume sous l'action du solvant pénétrant dans l'enceinte par osmose au fur et à mesure que les microorganismes fournissent le premier soluté.

Un inconvénient d'un tel actionneur et d'un tel moteur osmotique est que le maintien en vie des microorganismes impose des conditions d'utilisation contraignantes. Plus précisément, il est nécessaire de dissoudre dans le solvant dans lequel sont disposés les microorganismes des substances essentielles au métabolisme des microorganismes, par exemple le glucose et l'oxygène. Il est en outre nécessaire de prévoir l'évacuation des déchets produits par le métabolisme cellulaire, notamment le gaz carbonique. Par ailleurs, il est nécessaire de maintenir de nombreux paramètres tels que la température ou le pH du solvant dans lequel sont disposés les microorganismes dans des plages généralement très réduites en dehors desquelles les microorganismes ne peuvent survivre.

La présente invention vise un actionneur et un moteur osmotique dont la mise en oeuvre est simplifiée.

La présente invention vise également un actionneur et un moteur osmotique pouvant fonctionner sur une longue durée sans opération de maintenance contraignante.

La présente invention vise à utiliser, au lieu des microorganismes prévus dans la demande de brevet européen EP-A-1 481 165, un ou plusieurs catalyseurs adaptés à favoriser une réaction de transformation d'un composé en un autre composé. De tels catalyseurs peuvent correspondre par exemple à des enzymes qui sont des protéines douées d'un pouvoir catalytique très élevé. Par rapport à la demande de brevet EP-A-1481165, la présente invention se caractérise par une plus grande marge de manoeuvre quant aux conditions d'utilisation de l'actionneur et du moteur osmotique. En effet, les catalyseurs étant des composés non vivants, les contraintes visant à assurer leur intégrité sont moins importantes que celles visant la survie de microorganismes.

Plus particulièrement, la présente invention prévoit un actionneur comprenant une enceinte ayant une paroi non perméable à un premier soluté et perméable à un solvant et contenant, au moins temporairement, un catalyseur adapté à favoriser la transformation d'au moins un second soluté en le premier soluté pour faire varier la pression osmotique dans l'enceinte ; et une chambre déformable reliée à l'enceinte, ladite chambre étant adaptée à augmenter de volume sous l'action du solvant se déplaçant de l'enceinte dans la chambre par osmose ou ladite enceinte étant destinée à être disposée au contact du solvant, ladite chambre étant adaptée à augmenter de volume sous l'action du solvant pénétrant dans l'enceinte par osmose.

Selon un mode de réalisation de l'invention, ladite paroi de l'enceinte est perméable au second soluté.

Selon un mode de réalisation de l'invention, ladite paroi de l'enceinte est non perméable au second soluté, le catalyseur étant adapté à favoriser la transformation d'un nombre de particules du second soluté en un nombre plus élevé ou moins élevé de particules du premier soluté.

La présente invention prévoit également un moteur comprenant un actionneur tel que précédemment décrit, dans lequel la chambre comporte un moyen de rappel qui s'oppose à l'augmentation du volume de la chambre et un moyen commandable pour faire baisser la pression osmotique dans la chambre.

Selon un mode de réalisation de l'invention, la paroi de l'enceinte est non perméable au second soluté, le catalyseur étant adapté à favoriser la transformation d'un nombre de particules du second soluté en un nombre plus élevé de particules du premier soluté. Le moteur comprend en outre une enceinte supplémentaire ayant une paroi perméable au solvant et non perméable aux premier et second solutés et contenant un catalyseur supplémentaire adapté à favoriser la transformation d'un nombre de particules du premier soluté en un nombre plus faible de particules du second soluté, ladite enceinte supplémentaire étant reliée à la chambre par une vanne.

Selon un mode de réalisation de l'invention, la paroi de l'enceinte est non perméable au second soluté, le catalyseur étant adapté à favoriser la transformation d'un nombre de particules du second soluté en un nombre plus élevé de particules du premier soluté. L'enceinte est disposée dans une enveloppe déformable contenant le solvant et le premier soluté, l'enceinte contenant un catalyseur supplémentaire adapté à favoriser la transformation d'un nombre de particules du premier soluté en un nombre plus faible de particules du second soluté, le moyen pour faire baisser la pression osmotique dans la chambre étant une vanne adaptée à mettre en communication la chambre et l'enveloppe.

La présente invention prévoit également un moteur comprenant un actionneur tel que précédemment décrit, dans lequel l'enceinte est au moins en partie déformable et est reliée à la chambre au niveau de la paroi. Le moteur comprend un premier moyen de fourniture dans l'enceinte du catalyseur, et un second moyen de fourniture dans l'enceinte d'un catalyseur supplémentaire adapté à favoriser la transformation du premier soluté en le second soluté.

Selon un mode de réalisation de l'invention, la paroi est perméable au second soluté. Le catalyseur est adapté à favoriser la transformation d'un nombre de particules du second soluté en un nombre plus faible de particules du premier soluté et le catalyseur supplémentaire est adapté à favoriser la transformation d'un nombre de particules du premier soluté en un nombre plus élevé de particules du second soluté.

Selon un mode de réalisation de l'invention, le second soluté est un composé comprenant une fonction amine, le premier soluté étant un complexe du second soluté et d'un soluté supplémentaire comprenant une fonction aldéhyde, la paroi étant non perméable au soluté supplémentaire. En outre, le catalyseur est l'ion hydrogène, le catalyseur supplémentaire étant l'ion hydroxyle.

Selon un mode de réalisation de l'invention, le premier moyen de fourniture comprend une enceinte supplémentaire destinée à recevoir un solvant contenant du glucose, l'enceinte supplémentaire contenant des enzymes glucose oxydase adaptées à favoriser l'oxydation du glucose pour fournir des ions gluconate et des ions hydrogène.

Selon un mode de réalisation de l'invention, le second moyen de fourniture comprend une enceinte supplémentaire destinée à recevoir un solvant contenant de l'urée, l'enceinte supplémentaire contenant des enzymes uréase adaptées à favoriser l'oxydation de l'urée pour fournir des ions ammonium et du dioxyde de carbone.

La présente invention prévoit également une solution à osmolarité variable en fonction du pH comprenant une première substance ayant une fonction amine et une seconde substance ayant une fonction aldéhyde.

Selon un mode de réalisation de l'invention, la première substance est l'urée et la seconde substance est la vanilline.

Selon un mode de réalisation de l'invention, la seconde substance est un dérivé de la vanilline,

Ces objets, caractéristiques et avantages, ainsi que d'autres de la présente invention seront exposés en détail dans la description suivante de modes de réalisation particuliers faite à titre non-limitatif en relation avec les figures jointes parmi lesquelles :
les figures 1A et 1B représentent deux étapes de fonctionnement d'un premier mode de réalisation d'un moteur selon l'invention ;
les figures 2A et 2B représentent deux étapes de fonctionnement d'une variante du premier mode de réalisation du moteur selon l'invention ;
les figures 3A à 3C représentent trois étapes de fonctionnement d'un deuxième mode de réalisation du moteur selon l'invention ;
les figures 4A à 4C représentent trois étapes de fonctionnement d'un troisième mode de réalisation du moteur selon l'invention ;
les figures 5A à 5D représentent quatre étapes de fonctionnement d'un quatrième mode de réalisation du moteur selon l'invention ;
la figure 6 représente une variante du quatrième mode de réalisation ; et
la figure 7 représente une variante du quatrième mode de réalisation.

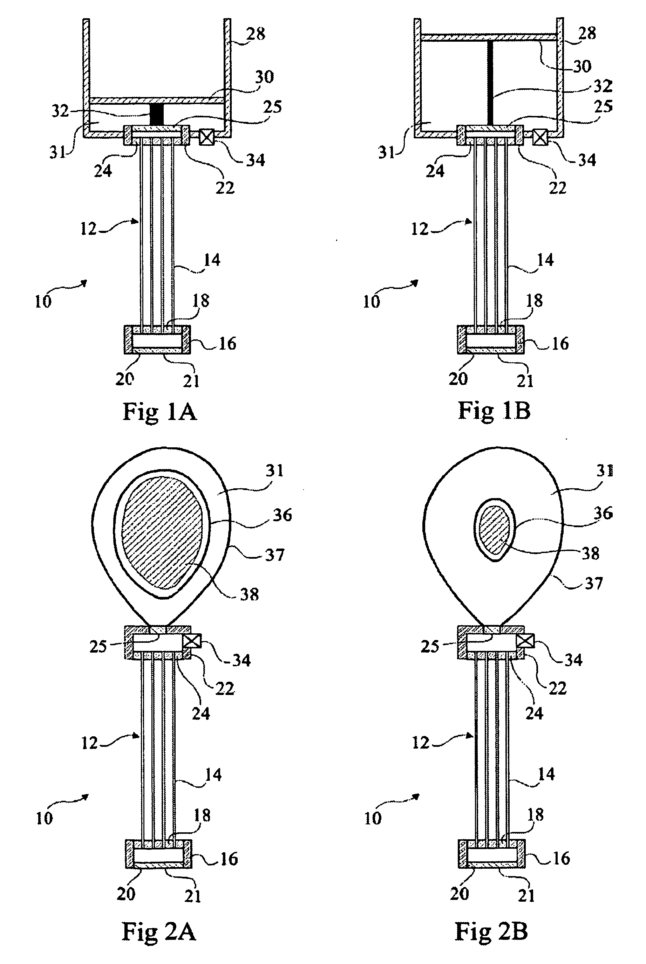

Sur les figures 1A et 1B, un moteur osmotique 10 selon l'invention comprend une enceinte 12 constituée par un faisceau de fibres creuses à paroi semi-perméables 14, par exemple du type utilisé pour les opérations de dialyse. La paroi des fibres 14 a un seuil de coupure donné, par exemple de l'ordre de 200 Daltons, c'est-à-dire qu'elle laisse passer des particules ayant une masse molaire sensiblement inférieure à 200 Daltons, c'est-à-dire à 200 g/mol. Chaque fibre a, par exemple, un diamètre de l'ordre de 200 µm. Le faisceau de fibres 14 est maintenu à une première extrémité par une première bague de jonction 16, par exemple par l'intermédiaire d'une zone de collage 18. La bague 16 comprend une ouverture 20 fermée par un bouchon 21. La seconde extrémité du faisceau de fibres 14 est maintenue par une seconde bague de jonction 22, par exemple par l'intermédiaire d'une zone de collage 24. Une membrane 25, ayant un seuil de coupure de l'ordre de 1000 Daltons, ferme la seconde bague 22.

L'enceinte 12 est fixée au niveau de la seconde bague de jonction 22, à une extrémité d'un corps cylindrique 28 dans lequel peut coulisser un piston mobile 30. Le piston mobile 30 et le corps cylindrique définissent une chambre de dilatation 31. Un moyen de rappel 32, par exemple un ressort, exerce sur le piston 30 un effort de traction tendant à le ramener dans une position de repos. Le corps cylindrique 28 comprend une vanne d'évacuation 34 communiquant avec l'extérieur du moteur 10.

On dispose à l'intérieur des fibres 14 un catalyseur adapté à favoriser une réaction de synthèse d'une substance X osmotiquement active à partir d'une substance Y, la substance Y ayant une masse molaire plus faible que la masse molaire de la substance X. Le seuil de coupure des fibres 14 est fixé pour empêcher le passage de la substance X et du catalyseur mais pour permettre le passage de la substance Y, tandis que le seuil de coupure de la membrane 25 est fixé pour permettre le passage de la substance X mais pour bloquer le catalyseur. En fonctionnement normal, le moteur 10 est placé dans un milieu ambiant comprenant un solvant dans lequel la substance Y est dissoute.

Un cycle de fonctionnement du moteur 10 se déroule alors de la façon suivante.

La figure 1A représente le moteur 10 au début d'un cycle. Le piston 30 est en position de repos, le volume de la chambre de dilatation 31 étant minimal, et la vanne d'évacuation 34 est fermée. Le catalyseur favorise la fabrication de la substance X à partir de la substance Y, ce qui tend à faire augmenter la pression osmotique à l'intérieur du faisceau de fibres 14. Le solvant du milieu ambiant pénètre dans les fibres 14 et dans la chambre de dilatation 31, déplaçant ainsi le piston 30. Le déplacement du piston 30 tend le ressort 32, permettant d'emmagasiner de l'énergie mécanique.

Sur la figure 1B, la chambre de dilatation 31 est représentée en expansion maximale. La vanne d'évacuation 34 s'ouvre alors. La pression à l'intérieur de la chambre de dilatation 31 s'égalise avec la pression du milieu ambiant. Le ressort 32 ramène le piston 30 en position de repos en évacuant, par la vanne d'évacuation 34, le solvant de la chambre de dilatation 31 dans le milieu ambiant. L'énergie mécanique emmagasinée dans le ressort 32 est ainsi récupérée. La vanne 34 est finalement fermée, achevant ainsi le cycle moteur.

Le piston 32 peut être relié à un élément extérieur auquel on souhaite transmettre une énergie mécanique.

A titre d'exemple, la substance X est un polymère de glucose comportant un nombre élevé de molécules de glucose, par exemple le dextrane, et la substance Y est un oligomère (qui, par définition, comprend un petit nombre d'unités monomères) de glucose de poids moléculaire minimum égal à 342 g/mol. Le catalyseur peut alors être une enzyme dextransucrase, c'est-à-dire une enzyme qui favorise la synthèse de dextrane à partir d'un oligomère de glucose.

Selon le premier mode de réalisation, la chambre de dilatation 31 est réalisée par un corps cylindrique dans lequel coulisse un piston. En fonction de l'utilisation souhaitée du moteur 10 selon l'invention, la chambre de dilatation 31 peut être réalisée de façon différente.

Les figures 2A et 2B représentent une variante de structure de la chambre de dilatation 31 du moteur 10 du premier mode de réalisation. Selon cette variante, la chambre de dilatation 31 correspond à l'espace défini entre une enveloppe intérieure 36 et une enveloppe extérieure 37 à la manière d'une chambre à air. L'enveloppe intérieure 36 est déformable et extensible et entoure un corps déformable 38. L'enveloppe extérieure 37 est souple et inextensible. Elle se referme sur l'enveloppe intérieure 36 et est reliée à la bague de jonction 22 de l'enceinte 12. La vanne d'évacuation 34 est disposée sur la bague de jonction 22. A titre d'exemple, dans une application médicale du moteur osmotique 10 selon l'invention, le corps déformable 38 peut être le coeur humain et les enveloppes peuvent définir des chambres de dilatation 31 en forme de boudins entourant le coeur.

Un cycle du moteur 10 selon la variante du premier mode de réalisation est le suivant.

La figure 2A représente le moteur 10 en début de cycle. Le volume de la chambre de dilatation 31 est minimal, le corps déformable 38 étant en dilation maximale, ce qui peut correspondre à un coeur en diastole. La vanne d'évacuation 34 est alors fermée. Le catalyseur favorise la formation de la substance X, ce qui entraîne, par osmose, l'introduction de solvant dans la chambre de dilatation 31. L'enveloppe intérieure 36 se déforme et comprime le corps déformable 38.

Sur la figure 2B, le corps déformable 38 est comprimé au maximum, ce qui peut correspondre à un coeur en systole. A l'ouverture de la vanne d'évacuation 34, le solvant évacue la chambre de dilatation 31 permettant la dilation du corps déformable 38, ce qui achève le cycle.

Selon une autre variante de l'invention, la chambre de dilatation est constituée d'une enveloppe élastique enfermant les fibres qui sont disposées, par exemple, en spirale, les deux bagues de jonction étant étanches. Lors de la synthèse de la substance osmotiquement active, les fibres ont tendance à se redresser et à déformer l'enveloppe élastique. On prévoit une vanne d'évacuation au niveau d'une bague de jonction. A l'ouverture de la vanne, la pression à l'intérieur des fibres diminue et l'enveloppe tend à reprendre sa forme initiale.

Selon une autre variante de l'invention, l'enceinte peut être reliée à la chambre de dilatation par un conduit souple. Ceci permet de disposer de façon avantageuse l'enceinte dans un milieu ambiant propice pour l'apport en solvant dans lequel est dissoute la substance Y, et de placer la chambre de dilatation à un endroit où l'on souhaite disposer de l'énergie mécanique. Dans le cas d'une application médicale, on pourra disposer l'enceinte dans un tissu graisseux, ou sur le réseau vasculaire. Dans ce dernier cas, les fibres peuvent être agencées pour former un tube creux, laissant en son centre un espace cylindrique permettant la circulation d'un fluide tel que le sang. Les bagues de jonction peuvent être de forme torique et placées contre la paroi d'un vaisseau sanguin. L'une des bagues de jonction toriques communique avec la chambre de dilatation par le conduit souple qui perfore le vaisseau sanguin.

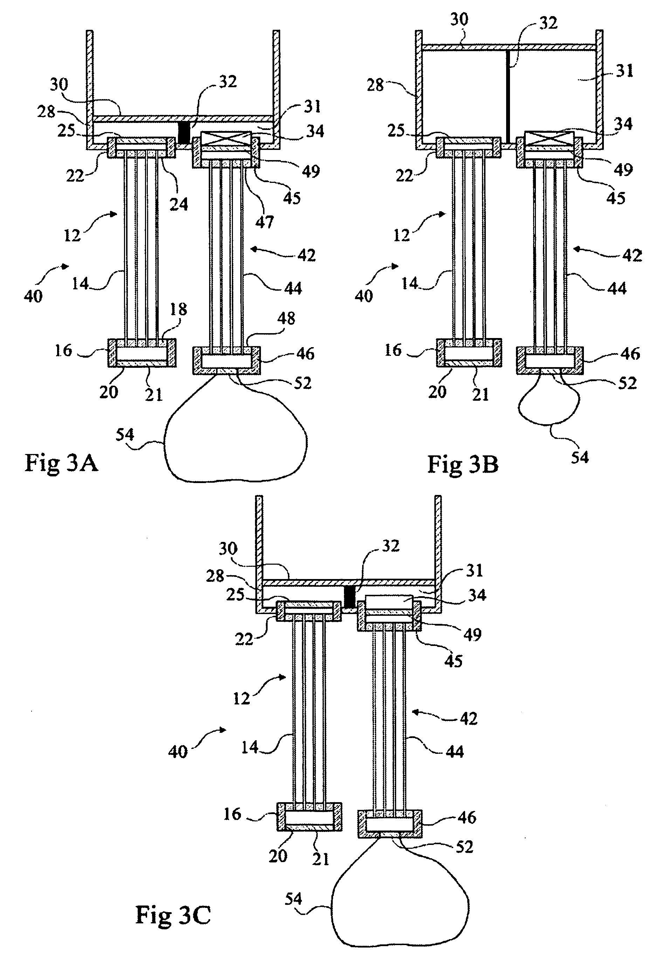

Les figures 3A à 3C représentent un deuxième mode de réalisation d'un moteur osmotique selon l'invention. Le moteur 40 inclut les composants du moteur du premier mode de réalisation et les références associées à celui-ci sont conservées.

Le moteur 40 comprend une première enceinte 12 du type décrit précédemment et une seconde enceinte 42. La seconde enceinte 42 comprend un second faisceau de fibres 44 maintenu aux extrémités par des bagues de jonction 45, 46 au moyen de zones de collage 47, 48. La seconde enceinte 42 est fixée sur le corps cylindrique 28 au niveau de la vanne 34, par la bague de jonction 45 qui comprend une membrane 49 séparant la chambre de dilatation 31 des secondes fibres 44. La seconde enceinte 42 communique, au niveau de la bague 46 par l'intermédiaire d'une membrane 52, avec un réservoir 54 déformable et étanche.

On dispose, dans le premier faisceau de fibres 14, un premier catalyseur C₁ favorisant la formation d'une substance Y à partir d'une substance X, de sorte qu'à partir d'une particule élémentaire de la substance X, il est produit plus d'une particule élémentaire de la substance Y. On dispose, dans le second faisceau de fibres 44, un second catalyseur C₂ favorisant la formation de la substance X à partir de la substance Y, de sorte que pour produire une particule élémentaire de la substance X, il est utilisé plus d'une particule élémentaire de la substance Y.

Selon un exemple, la substance X est un polymère du glucose comportant un nombre élevé de molécules de glucose, par exemple le dextrane, et la substance Y est un oligomère de glucose. Le second catalyseur C₂ peut alors être une enzyme dextransucrase, c'est-à-dire une enzyme qui favorise la synthèse de dextrane à partir d'un oligomère de glucose et le premier catalyseur C₁ peut alors être une enzyme dextranase, c'est-à-dire une enzyme qui favorise la dégradation du dextrane en glucose ou en oligomères de glucose.

Les parois des faisceaux de fibres 14, 44 ont un seuil de coupure inférieur à la masse molaire des substances X et Y. A titre d'exemple, le seuil de coupure est de l'ordre de 100 Daltons lorsque la substance Y est du glucose ou un oligomère de glucose et la substance X est du dextrane. Les membranes 25, 49 et 52 ont des seuils de coupure supérieurs à 1000 Daltons, de façon à laisser passer les substances X et Y et maintenir les catalyseurs dans les faisceaux de fibres respectifs 14, 44.

En fonctionnement normal, le moteur 40 est placé dans un solvant. Le cycle de fonctionnement du moteur osmotique 40 selon l'invention est le suivant.

La figure 3A représente le moteur 40 au début du cycle. La vanne 34 est fermée. Les concentrations de la substance Y sont identiques dans le réservoir 54 et la chambre de dilatation 31, de même que les concentrations de la substance X. Dans le premier faisceau de fibres 14, le catalyseur C₁ favorise la formation de la substance Y, ce qui augmente la pression osmotique dans la chambre de dilatation 31. Le solvant pénètre dans le premier faisceau de fibres 14 puis dans la chambre de dilatation 31, déplaçant ainsi le piston 30 et emmagasinant de l'énergie mécanique par la mise en tension du ressort 32. Pendant ce temps, dans le second faisceau de fibres 44, le catalyseur C₂ favorise la formation de la substance X, ce qui diminue la pression osmotique dans le réservoir 54. Le réservoir 54 diminue de volume, sans produire de travail utile puisque rien ne s'oppose à cette diminution.

La figure 3B représente le moteur 10 à la fin de l'étape décrite précédemment, la chambre de dilatation 31 ayant un volume maximum.

La vanne 34 s'ouvre alors. Les concentrations en substance X et en substance Y s'équilibrent dans les faisceaux de fibres 14, 44, la chambre de dilatation 31 et le réservoir 54. De même, les pressions osmotiques s'équilibrent dans les différents compartiments. Le piston 30 descend donc sous l'action du ressort 32 jusqu'à la position représentée sur la figure 3C. De plus, le réservoir 54 se dilate en se remplissant de liquide, le travail nécessaire pour dilater le réservoir 54 étant négligeable devant celui procuré par le ressort 32, les pressions dans le milieu ambiant étant faibles devant celles présentes dans la chambre de dilatation 31. La vanne 34 est alors refermée, ce qui clôt le cycle.

Le deuxième mode de réalisation est particulièrement avantageux dans la mesure où les échanges entre le moteur 40 et le milieu ambiant sont réduits par rapport au premier mode de réalisation. En effet, dans le premier mode de réalisation, la substance X osmotiquement active, par exemple le dextrane, est produite à partir de la substance Y, par exemple un oligomère de glucose, présent dans le solvant. En outre, à la fin d'un cycle moteur, la vanne d'évacuation 34 est ouverte et la majeure partie de la substance X formée est libérée dans le milieu ambiant. Dans le cas d'une application médicale, la substance X produite, par exemple le dextrane, est libérée dans le corps humain, ce qui peut être problématique. Dans le second mode de réalisation, il y a juste transfert de solvant entre le moteur 40 et le milieu ambiant.

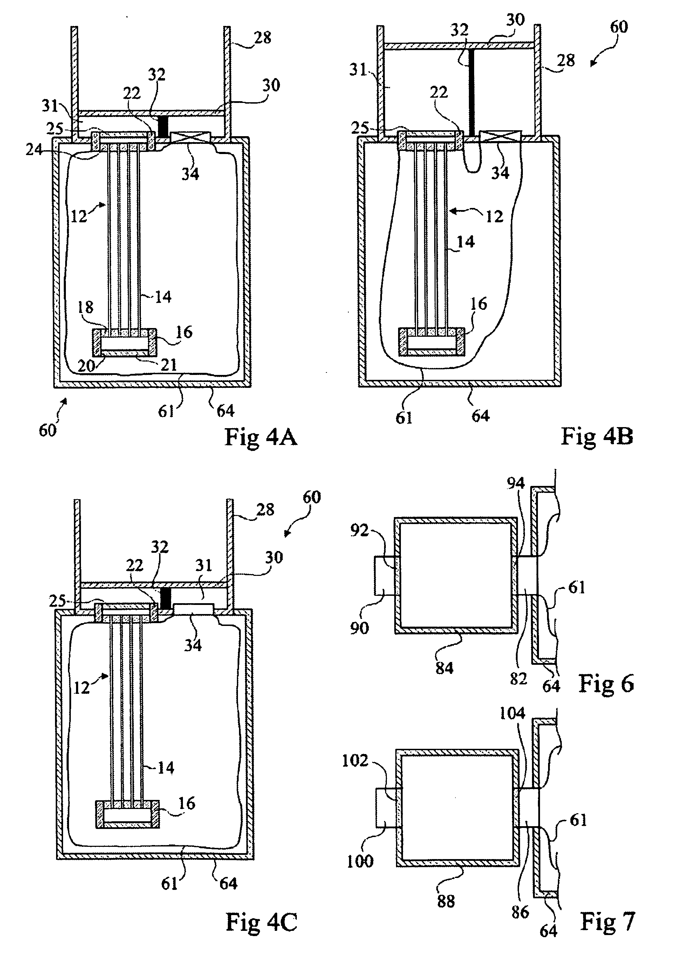

Les figures 4A à 4C représentent un troisième mode de réalisation du moteur osmotique 60 selon l'invention. Le moteur 60 inclut les composants du moteur 10 du premier mode de réalisation et les références associées à celui-ci sont conservées.

L'enceinte 12 est disposée dans une enveloppe 61 déformable et étanche qui se referme sur la bague de jonction 22 et la vanne d'évacuation 34. L'enveloppe 61 est remplie d'un solvant. L'enveloppe 61 peut être disposée dans un carter rigide 64 perforé pour ne pas empêcher les déformations de l'enveloppe 61.

On dispose, dans le faisceau de fibres 14 de l'enceinte 12, un premier catalyseur C₁ favorisant la formation d'une substance Y (par exemple un oligomère de glucose) à partir d'une substance X (par exemple du dextrane), de sorte qu'à partir d'une particule élémentaire de X, on produit plus d'une particule élémentaire de Y. On dispose, dans l'enveloppe 61, un second catalyseur C₂ favorisant la formation de la substance X à partir de la substance Y, de sorte que pour produire une particule élémentaire de la substance X, on utilise plus d'une particule élémentaire de la substance Y.

Les membranes du faisceau de fibres 14 ont un seuil de coupure inférieur à la masse molaire des substances X et Y. A titre d'exemple, le seuil de coupure est de l'ordre de 100 Daltons lorsque la substance Y est du glucose ou un oligomère de glucose, et la substance X du dextrane.

Un cycle de fonctionnement du moteur 60 selon le troisième mode de réalisation est le suivant.

La figure 4A représente le moteur 60 au début d'un cycle. La vanne 34 est fermée. L'enveloppe 61 est au maximum de son volume. Tous les compartiments contiennent un solvant où sont en solution les substances X et Y. Les concentrations en X sont sensiblement équilibrées entre l'enveloppe 61 et la chambre de dilatation 31. Le premier catalyseur C₁ favorise la formation de la substance Y, ce qui augmente la pression osmotique dans le faisceau 14 et la chambre de dilatation 31. Le second catalyseur C₂ favorise la formation de la substance X, réduisant la pression osmotique à l'intérieur de l'enveloppe 61. Des échanges de solvant se produisent, le solvant passant vers le faisceau de fibres 14 et, de là, vers la chambre de dilatation 31 entraînant le déplacement du piston 30. Le piston est en phase ascendante.

La figure 4B représente le moteur 60 à la fin de l'étape précédemment décrite. La concentration en substance X est minimale dans le faisceau de fibres 14 et maximale à l'intérieur de l'enveloppe 61. A l'inverse, la concentration en substance Y est maximale dans le faisceau de fibres 14 et minimale dans l'enveloppe 61.

On ouvre ensuite la vanne d'évacuation 34 qui met en communication directe la chambre de dilatation 31 et l'enveloppe 61. La pression dans la chambre de dilatation 31 s'effondre et le ressort de rappel 32 ramène le piston 30 en position initiale évacuant le solvant de la chambre de dilatation 31 vers l'enveloppe 61. Le piston est dit en phase descendante. Le faisceau de fibres 14 est ainsi mis en communication avec l'intérieur de l'enveloppe 61. Les concentrations en substances X et Y s'égalisent entre ces deux compartiments.

La figure 4C représente le moteur 60 à la fin de la phase descendante du piston 30. La vanne 34 est ensuite fermée, ce qui clôt le cycle.

Une variante du troisième mode de réalisation du moteur osmotique peut être utilisée comme moteur pour l'entraînement des roues d'un véhicule automobile. Selon cette variante, le ressort est supprimé et le piston est relié, par exemple, par une bielle à un arbre moteur d'entraînement des roues de façon analogue à la liaison entre un piston d'un moteur thermique et le vilebrequin. La force motrice correspond à la phase ascendante du piston, c'est-à-dire à la phase d'expansion de la chambre de dilatation. En phase descendante, lorsque le volume de la chambre de dilatation diminue, le piston ne rencontre qu'une faible résistance, correspondant au passage du solvant au travers de la vanne d'évacuation de la chambre de dilatation. De façon avantageuse, au moins deux moteurs osmotiques peuvent être mis en parallèle pour entraîner l'arbre moteur de sorte que les chambres de dilatation des moteurs travaillent en opposition, l'une étant en phase d'expansion lorsque l'autre est en phase de contraction.

Un tel moteur peut, en outre, être utilisé pour récupérer de l'énergie lors du freinage du véhicule. Dans ce cas, il comporte une vanne supplémentaire, appelée vanne d'alimentation disposée entre le premier faisceau de fibres et la chambre de dilatation. Lors du fonctionnement du moteur pour l'entraînement des roues, la vanne d'alimentation est ouverte de sorte que le moteur fonctionne comme cela a été précédemment décrit.

Dans le cas d'un freinage, quand le piston est en phase ascendante, la vanne d'alimentation est fermée et la vanne d'évacuation est ouverte de sorte que la chambre de dilatation se remplit de liquide sans effort significatif. Une grande partie du liquide contenu dans l'enveloppe déformable passe dans la chambre de dilatation sans que les concentrations dans les divers compartiments varient. La vanne d'alimentation est ensuite ouverte et la vanne d'évacuation fermée. Lorsque le piston descend sous l'action de l'arbre moteur entraîné par les roues, le liquide contenu dans la chambre de dilatation est chassé au travers du faisceau de fibres et arrive dans l'enveloppe. Le travail ainsi produit permet à la fois de ralentir le véhicule, mais également de faire varier les concentrations des substances X et Y. En effet, l'osmolarité du faisceau de fibres augmente, alors que l'osmolarité dans l'enveloppe déformable diminue.

De ce fait, lorsque le moteur fonctionne à nouveau comme moteur d'entraînement des roues, la vanne d'alimentation étant ouverte, le cycle moteur reprend avec une efficacité supérieure. En effet, les différences de concentrations entre les compartiments vont créer une différence de pression grâce à laquelle le cycle se réalisera plus rapidement.

En outre, les enceintes 12 et 42 peuvent être réalisées autrement que par un faisceau de fibres. Elles peuvent avoir toute forme permettant un bon échange des solutés et du solvant de part et d'autre de la paroi de l'enceinte.

De plus, la vanne 34 peut présenter tout type de structure connue. Les ouvertures et fermetures de la vanne peuvent par exemple être commandées par un dispositif externe au moteur, de façon synchrone ou non, ou bien être déclenchées automatiquement par la structure même de la vanne lorsque la pression dans un compartiment du moteur excède une valeur déterminée.

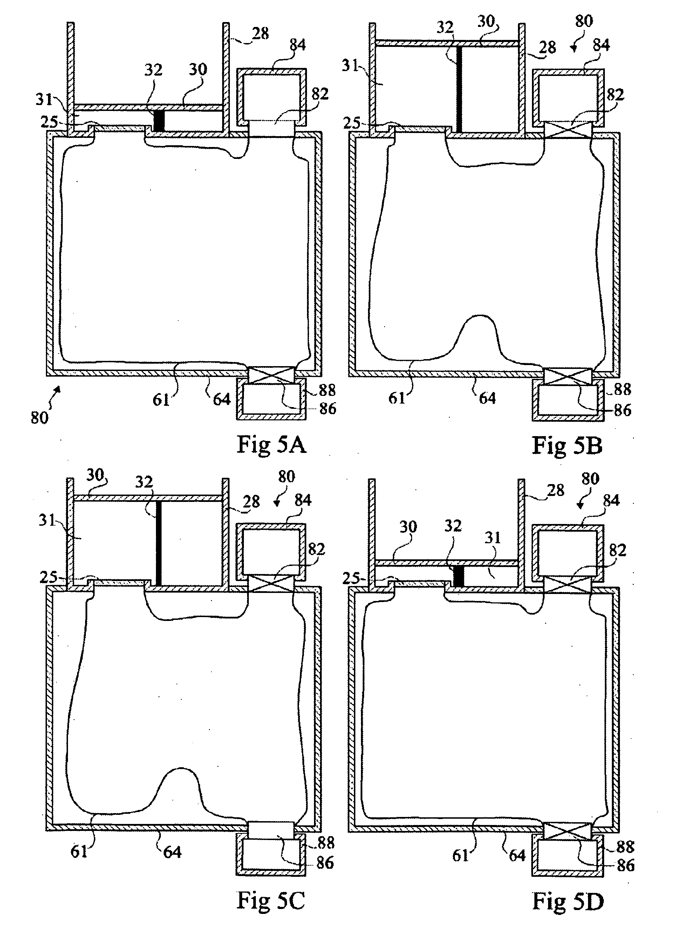

Les figures 5A à 5D représentent un quatrième mode de réalisation du moteur osmotique 80 selon l'invention. Le moteur 80 inclut certains composants du moteur 60 du troisième mode de réalisation et les références associées à celui-ci sont conservées.

Par rapport au moteur 60, l'enceinte 12 est supprimée. Une vanne 82 est disposée au niveau du carter rigide 64 et est adaptée à faire communiquer, lorsqu'elle est ouverte, le contenu de l'enveloppe 61 avec le contenu d'une enceinte 84. Une vanne 86 est disposée au niveau du carter rigide 64 et est adaptée à faire communiquer, lorsqu'elle est ouverte, le contenu de l'enveloppe 61 avec le contenu d'une enceinte 88.

L'enveloppe 61 contient des substances U et V, dissoutes dans un solvant et qui sont susceptibles de réagir en présence d'ions H⁺, c'est-à-dire à un pH suffisamment acide, pour former une substance Z. L'ion H⁺ joue un rôle similaire à celui d'un catalyseur de la réaction de synthèse de la substance Z. La réaction inverse selon laquelle la substance Z est dégradée pour redonner les substances U et V est susceptible de se produire en présence d'ions OH⁻, c'est-à-dire à un pH suffisamment basique. L'ion OH- joue un rôle similaire à celui d'un catalyseur de la réaction de dégradation de la substance Z. La membrane 25 a un seuil de coupure suffisamment faible pour bloquer les substances U et V qui sont donc osmotiquement actives. Les vannes 82, 86 sont associées à des membranes, non représentées, qui ont un seuil de coupure tel qu'elles bloquent les substances U et V. Selon le quatrième mode de réalisation, l'enceinte 84 contient une solution acide et l'enceinte 88 contient une solution basique.

Les substances U, V et Z doivent être solubles dans le solvant, et si possible biocompatibles dans le cas d'une application médicale. A titre d'exemple, la substance U est une molécule comprenant une fonction amine primaire (fonction -NH₂) et la substance V est une molécule comprenant une fonction aldéhyde (fonction -CHO). Des réactions de formation de complexes à partir d'une substance U à fonction amine et d'une substance V à fonction aldéhyde sont décrites dans les ouvrages "Advanced Organic Chemistry: Reactions, Mechanisms and Structure", troisième édition, J. March, John Wilez and Sons, New York (1985), PP 796-798 et "Advanced Organic Chemistry, Part B: Reactions and Synthesis", troisième édition, F.A. Carey et R.J. Sundberg, Plenum Press, New-York (1990), pp 30-31. A titre d'exemple, la substance U peut être l'urée de masse molaire 60 g/mol, et la substance V peut être la vanilline de masse molaire 152 g/mol. La membrane 25 a alors un seuil de coupure de 50 Daltons. La substance Z correspond à un complexe urée-vanilline. Dans ce cas, la réaction de formation du complexe urée-vanilline est favorisée pour un pH inférieur à 6 tandis que la réaction de dégradation du complexe urée-vanilline (qui redonne de l'urée et de la vanilline) est favorisée pour un pH de l'ordre de 8. Selon un autre exemple, le composé V est un dérivé de la vanilline ayant la formule suivante : 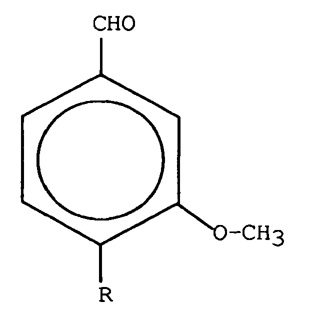 où R est un groupe carboné (R correspondant au groupe hydroxyle -OH dans le cas de la vanilline).

La chambre de dilatation 31 contient une substance A, dissoute dans le solvant, osmotiquement active. La membrane 25 a un seuil de coupure suffisamment faible pour bloquer la substance A. A titre d'exemple, la substance A est le dextrane.

Un cycle de fonctionnement du moteur 80 selon le quatrième mode de réalisation est le suivant.

La figure 5A représente le moteur 80 au début d'un cycle. L'enveloppe 61 est au maximum de son volume. Les concentrations des substances U et V et la concentration de la substance A sont initialement choisies de sorte que les pressions osmotiques dans la chambre de dilatation 31 et dans l'enveloppe 61 s'équilibrent, le piston 30 restant fixe.

La vanne 86 est fermée et la vanne 82 est alors ouverte. Les ions H⁺ se répandent dans l'enveloppe 61 entraînant une diminution du pH. Lorsque le pH dans l'enveloppe 61 a diminué jusqu'à la valeur souhaitée, la vanne 82 est fermée. La réaction de formation de la substance Z à partir des substances U et V est alors favorisée. La pression osmotique dans l'enveloppe 61 diminue par rapport à la pression osmotique dans la chambre de dilatation 31 qui ne varie pas. Un transfert de solvant se produit donc, du solvant passant de l'enveloppe 61 à la chambre de dilatation 31 en traversant la membrane 25, entraînant le déplacement du piston 30. Le piston 30 est en phase ascendante.

La figure 5B représente le moteur 80 à la fin de la phase ascendante du piston 30.

En figure 5C, on a ouvert la vanne 86 de façon à mettre en communication le contenu de l'enveloppe 61 avec le contenu de l'enceinte 88. Des ions OH- se répandent dans l'enveloppe 61 entraînant une augmentation du pH. Lorsque le pH est suffisamment basique, la vanne 86 est fermée. La réaction de dégradation de la substance Z est alors favorisée, entraînant l'augmentation du nombre de particules osmotiquement actives dans l'enveloppe 61. La pression osmotique augmentant dans l'enveloppe 61, un nouveau transfert de solvant se produit, du solvant passant de la chambre de dilatation 31 vers l'enveloppe 61 par l'intermédiaire de la membrane 25. L'action du ressort 32 favorise l'évacuation du solvant de la chambre de dilatation. Toutefois, le ressort 32 pourrait ne pas être présent, le piston se déplaçant par succion. Le piston 30 est dit en phase descendante.

La figure 5D représente le moteur 80 à la fin de la phase descendante du piston 30, ce qui clôt le cycle. Les concentrations des substances U et V dans l'enveloppe 61 sont alors sensiblement identiques aux concentrations en début de cycle.

Selon une variante du quatrième mode de réalisation, la membrane 25 est seulement imperméable à la substance V mais perméable à la substance U. Dans ce cas, seuls les substances A, Z et V sont osmotiquement actives. La substance U est libre de diffuser à travers la membrane 25 et n'est donc pas osmotiquement active. Dans le cas où la substance U est l'urée et la substance V est la vanilline, la membrane 25 a par exemple un seuil de coupure de l'ordre de 100 Daltons.

Les substances U et V ont alors la caractéristique supplémentaire que la formation d'une particule de la substance Z utilise, au moins en moyenne, plus d'une particule de la substance V. Ceci est vérifié lorsque la substance U est l'urée et la substance V est la vanilline. En effet, une molécule du complexe urée-vanilline est en moyenne obtenue à partir d'une molécule d'urée et plus d'une molécule de vanilline (une ou deux molécules de vanilline venant se greffer sur l'urée selon l'encombrement stérique). Une telle réaction de substitution est décrite dans la référence précédemment citée "Advanced Organic Chemistry: Reactions, Mechanisms and Structure", troisième édition, J. March, John Wilez and Sons, New York (1985), pp 798.

Dans ce cas, un cycle de fonctionnement du moteur 80 est le suivant.

Au début d'un cycle (figure 5A), l'enveloppe 61 est au maximum de son volume. La concentration de la substance V et la concentration de la substance A sont initialement choisies de sorte que les pressions osmotiques dans la chambre de dilatation 31 et dans l'enveloppe 61 s'équilibrent, le piston 30 restant fixe.

La vanne 86 est fermée et la vanne 82 est alors ouverte. Les ions H⁺ se répandent dans l'enveloppe 61 entraînant une diminution du pH dans l'enveloppe 61. La réaction de formation de la substance Z à partir des substances U et V est alors favorisée, la substance U présente dans la chambre de dilatation 31 passant dans l'enveloppe 61 pour participer à la réaction. La formation d'une particule de la substance Z nécessitant plus d'une particule de la substance V, le nombre de particules osmotiquement actives diminue dans l'enveloppe 61 par rapport à la chambre de dilatation 31. La pression osmotique dans l'enveloppe 61 diminue donc par rapport à la pression osmotique dans la chambre de dilatation 31. Un transfert de solvant se produit donc, du solvant passant de l'enveloppe 61 à la chambre de dilatation 31 en traversant la membrane 25, entraînant le déplacement du piston 30. Le piston 30 est en phase ascendante.

Lorsque la vanne 86 est ouverte, la vanne 82 étant fermée (figure 5C), et que le pH dans l'enveloppe 61 devient suffisamment basique pour favoriser la réaction de dégradation de la substance Z, le nombre de particules osmotiquement actives dans l'enveloppe 61 augmente (même si une partie de la substance U issue de la dégradation diffuse dans la chambre de dilatation 31). La pression osmotique augmentant dans l'enveloppe 61, un nouveau transfert de solvant se produit, du solvant passant de la chambre de dilatation 31 à l'enveloppe 61 en traversant la membrane 25.

Dans les exemples précédemment décrits, il est nécessaire de régulièrement recharger les enceintes 84, 88, respectivement en solution acide et basique.

La figure 6 représente une variante d'obtention de la solution acide contenue dans l'enceinte 84 adaptée au cas où l'enceinte 84 baigne dans un milieu ambiant liquide dans lequel est dissous du glucose, par exemple un solvant biologique. L'enceinte 84 comprend une vanne 90 adaptée à mettre en communication le contenu de l'enceinte 84 avec le milieu ambiant. Selon une telle variante, on favorise dans l'enceinte 84 la réaction d'oxydation du glucose (noté R-COH) en gluconate (noté R-COO⁻) selon la réaction suivante :

R-COH + 1/2 O₂-> RCOO⁻ + H⁺

Une telle réaction est rendue possible en prévoyant dans l'enceinte 84 une enzyme glucose oxydase. On prévoit alors une membrane 92 entre la vanne 90 et l'enceinte 84 et une membrane 94 entre la vanne 82 et l'enceinte 64, les membranes 92, 94 permettant de retenir l'enzyme glucose oxydase, la membrane 92 laissant passer le glucose.

Pour obtenir une solution acide dans l'enceinte 84, on ferme la vanne 82 et on ouvre la vanne 90 de façon que du glucose pénètre dans l'enceinte 84. La vanne 90 est alors fermée. Le glucose est alors décomposé en produisant des ions H⁺ et du gluconate. La solution contenue dans l'enceinte 84 devient donc acide et peut alors être utilisée au cours d'un cycle de fonctionnement du moteur 80. A la prochaine ouverture de la vanne 90, le gluconate diffuse hors de l'enceinte 84. Dans le cas d'une application médicale, le rejet de gluconate dans le corps humain ne présente pas de danger puisqu'il est naturellement évacué par les reins.

La figure 6 représente une variante d'obtention de la solution acide contenue dans l'enceinte 84 adaptée au cas où l'enceinte 84 baigne dans un milieu ambiant liquide dans lequel est dissous du glucose, par exemple un solvant biologique.

La figure 7 représente une variante d'obtention de la solution basique contenue dans l'enceinte 88 adaptée au cas où l'enceinte 88 baigne dans un milieu ambiant liquide dans lequel est dissous de l'urée, par exemple un solvant biologique. L'enceinte 88 comprend une vanne 100 adaptée à mettre en communication le contenu de l'enceinte 88 avec le milieu ambiant. Selon une telle variante, on favorise dans l'enceinte 88 la réaction de dégradation de l'urée en ammoniac et acide carbonique selon la réaction suivante :

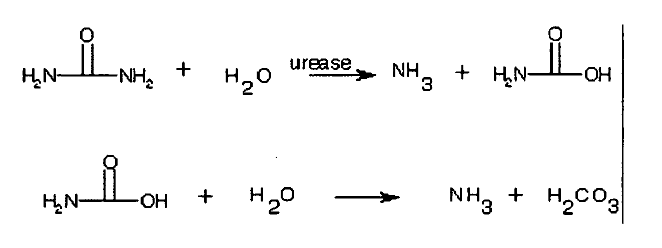

Au pH physiologique, l'acide carbonique se dissocie en eau et dioxyde de carbone. L'ammoniac va s'équilibrer avec l'eau pour devenir l'ion ammonium (NH₄⁺) résultant ainsi en une nette augmentation du pH. Le dioxyde de carbone sera naturellement évacué par la respiration.

Une telle réaction est rendue possible en prévoyant dans l'enceinte 88 une enzyme uréase. On prévoit alors une membrane 102 entre la vanne 100 et l'enceinte 88 et une membrane 104 entre la vanne 86 et l'enceinte 64, les membranes 102, 104 permettant de retenir l'enzyme uréase, la membrane 102 laissant passer l'ammoniac et le dioxyde de carbone.

Selon une autre variante de réalisation, dans le cas d'une application médicale les substances U, V et Z sont telles que la dégradation de la substance Z qui redonne les substances U et V est favorisée dès que le pH est faiblement basique, par exemple de l'ordre de 7,4. L'enceinte 88 contenant la solution basique et la vanne 86 sont utilisées pour mettre directement en communication le contenu de l'enveloppe 61 avec le milieu biologique ambiant. En effet, le liquide biologique humain est naturellement à un pH légèrement basique de l'ordre de 7,4.

Bien entendu, la présente invention est susceptible de diverses variantes et modifications qui apparaîtront à l'homme de l'art. En particulier, pour les deuxième et troisième modes de réalisation du moteur, la chambre de dilatation peut être réalisée selon les variantes décrites du premier mode de réalisation.

## Revendications

1. Actionneur comprenant :
une enceinte (12 ; 61) ayant une paroi (14 ; 25) non perméable à un premier soluté et perméable à un solvant et contenant, au moins temporairement, un catalyseur adapté à favoriser la transformation d'au moins un second soluté en le premier soluté pour faire varier la pression osmotique dans l'enceinte ; et
une chambre déformable (31) reliée à l'enceinte, ladite chambre étant adaptée à augmenter de volume sous l'action du solvant se déplaçant de l'enceinte dans la chambre par osmose ou ladite enceinte étant destinée à être disposée au contact du solvant, ladite chambre étant adaptée à augmenter de volume sous l'action du solvant pénétrant dans l'enceinte par osmose.

2. Actionneur selon la revendication 1, dans lequel ladite paroi (14 ; 25) de l'enceinte (12 ; 61) est perméable au second soluté.

3. Actionneur selon la revendication 1, dans lequel ladite paroi (14 ; 25) de l'enceinte (12 ; 61) est non perméable au second soluté, le catalyseur étant adapté à favoriser la transformation d'un nombre de particules du second soluté en un nombre plus élevé ou moins élevé de particules du premier soluté.

4. Moteur (10, 40, 60) comprenant un actionneur selon la revendication 1, dans lequel la chambre (31) comporte un moyen de rappel (32) qui s'oppose à l'augmentation du volume de la chambre et un moyen commandable (34) pour faire baisser la pression osmotique dans la chambre.

5. Moteur (40) selon la revendication 4, dans lequel ladite paroi (14) de l'enceinte (12) est non perméable au second soluté, le catalyseur étant adapté à favoriser la transformation d'un nombre de particules du second soluté en un nombre plus élevé de particules du premier soluté, ledit moteur comprenant en outre une enceinte supplémentaire (42) ayant une paroi (44) perméable au solvant et non perméable aux premier et second solutés et contenant un catalyseur supplémentaire adapté à favoriser la transformation d'un nombre de particules du premier soluté en un nombre plus faible de particules du second soluté, ladite enceinte supplémentaire étant reliée à la chambre (61) par une vanne (34).

6. Moteur (60) selon la revendication 4, dans lequel ladite paroi (14) de l'enceinte (12) est non perméable au second soluté, le catalyseur étant adapté à favoriser la transformation d'un nombre de particules du second soluté en un nombre plus élevé de particules du premier soluté et dans lequel l'enceinte (12) est disposée dans une enveloppe déformable (61) contenant le solvant et le premier soluté, l'enceinte (61) contenant un catalyseur supplémentaire adapté à favoriser la transformation d'un nombre de particules du premier soluté en un nombre plus faible de particules du second soluté, le moyen (34) pour faire baisser la pression osmotique dans la chambre étant une vanne (34) adaptée à mettre en communication la chambre (31) et l'enveloppe.

7. Moteur (80) comprenant un actionneur selon la revendication 1, dans lequel l'enceinte (61) est au moins en partie déformable et est reliée à la chambre (31) au niveau de la paroi (25), le moteur comprenant un premier moyen (82, 84) de fourniture dans l'enceinte du catalyseur, et un second moyen (86, 88) de fourniture dans l'enceinte d'un catalyseur supplémentaire adapté à favoriser la transformation du premier soluté en le second soluté.

8. Moteur selon la revendication 7, dans lequel la paroi (25) est perméable au second soluté, le catalyseur étant adapté à favoriser la transformation d'un nombre de particules du second soluté en un nombre plus faible de particules du premier soluté et le catalyseur supplémentaire étant adapté à favoriser la transformation d'un nombre de particules du premier soluté en un nombre plus élevé de particules du second soluté.

9. Moteur selon la revendication 7, dans lequel le second soluté est un composé comprenant une fonction amine, le premier soluté étant un complexe du second soluté et d'un soluté supplémentaire comprenant une fonction aldéhyde, la paroi (25) étant non perméable au soluté supplémentaire et dans lequel le catalyseur est l'ion hydrogène, le catalyseur supplémentaire étant l'ion hydroxyle.

10. Moteur selon la revendication 9, dans lequel le premier moyen de fourniture (82, 84) comprend une enceinte supplémentaire (84) destinée à recevoir un solvant contenant du glucose, l'enceinte supplémentaire contenant des enzymes glucose oxydase adaptées à favoriser l'oxydation du glucose pour fournir des ions gluconate et des ions hydrogène.

11. Moteur selon la revendication 9, dans lequel le second moyen de fourniture (86, 88) comprend une enceinte supplémentaire (88) destinée à recevoir un solvant contenant de l'urée, l'enceinte supplémentaire contenant des enzymes uréase adaptées à favoriser l'oxydation de l'urée pour fournir des ions ammonium et du dioxyde de carbone.

## Claims

1. An actuator comprising:
an enclosure (12; 61) having a wall (14; 25) impermeable to a first solute and permeable to a solvent and containing, at least temporarily, a catalyst capable of promoting the transformation of at least one second solute into the first solute to vary the osmotic pressure in the enclosure; and
a deformable chamber (31) connected to the enclosure, said chambre being capable of increasing in volume under the action of the solvent moving from the enclosure into the chamber by osmosis or said enclosure being designed to be arranged in contact with the solvent, said chamber being capable of increasing in volume under the action of the solvent penetrating into the enclosure by osmosis.

2. The actuator of claim 1, wherein said wall (14; 25) of the enclosure (12; 61) is permeable to the second solute.

3. The actuator of claim 1, wherein said wall (14; 25) of the enclosure (12; 61) is impermeable to the second solute, the catalyst being capable of promoting the transformation of a number of particles of the second solute into a greater or smaller number of particles of the first solute.

4. A motor (10, 40, 60) comprising the actuator of claim 1, wherein the chamber (31) comprises return means (32) which oppose to the volume increase of the chamber and controllable means (34) for lowering the osmotic pressure in the chamber.

5. The motor (40) of claim 4, wherein said wall (14) of the enclosure (12) is impermeable to the second solute, the catalyst being capable of promoting the transformation of a number of particles of the second solute into a greater number of particles of the first solute, said motor further comprising an additional enclosure (42) having a wall (44) permeable to the solvent and impermeable to the first and second solutes and containing an additional catalyst capable of promoting the transformation of a number of particles of the first solute into a smaller number of particles of the second solute, said additional enclosure being connected to the chamber (61) by a valve (34).

6. The motor (60) of claim 4, wherein said wall (14) of the enclosure (12) is impermeable to the second solute, the catalyst being capable of promoting the transformation of a number of particles of the second solute into a greater number of particles of the first solute and wherein the enclosure (12) is arranged in a deformable envelope (61) containing the solvent and the first solute, the enclosure (61) containing an additional catalyst capable of promoting the transformation of a number of particles of the first solute into a smaller number of particles of the second solute, the means (34) for lowering the osmotic pressure in the chamber being a valve (34) capable of connecting up the chamber (31) and the envelope.

7. A motor (80) comprising the actuator of claim 1, wherein the enclosure (61) is at least partly deformable and is connected to the chamber (31) at the level of the wall (25), the motor comprising first means (82, 84) for supplying the catalyst into the enclosure, and second means (86, 88) for supplying an additional catalyst, capable of promoting the transformation of the first solute into the second solute, into the enclosure.

8. The motor of claim 7, wherein the wall (25) is permeable to the second solute, the catalyst being capable of promoting the transformation of a number of particles of the second solute into a smaller number of particles of the first solute and the additional catalyst being capable of promoting the transformation of a number of particles of the first solute into a greater number of particles of the second solute.

9. The motor of claim 7, wherein the second solute is a compound comprising an amine function, the first solute being a complex of the second solute and of an additional solute comprising an aldehyde function, the wall (25) being impermeable to the additional solute and wherein the catalyst is the hydrogen ion, the additional catalyst being the hydroxyl ion.

10. The motor of claim 9, wherein the first supply means (82, 84) comprise an additional enclosure (84) designed to receive a solvent containing glucose, the additional enclosure containing glucose oxidase enzymes capable of promoting the oxidation of glucose to provide gluconate ions and hydrogen ions.

11. The motor of claim 9, wherein the second supply means (86, 88) comprise an additional enclosure (88) designed to receive a solvent containing urea, the additional enclosure containing urease enzymes capable of promoting the oxidation of urea to provide ammonium ions and carbon dioxide.

## Patentansprüche

1. Ein Stellglied bzw. Aktuator, der Folgendes aufweist:
eine Umhüllung (12; 61) mit einer Wand (14; 25), die für einen ersten gelösten Stoff (solute) impermeabel ist und für ein Lösungsmittel permeabel ist und die, zumindest zeitweise, einen Katalysator enthält, der in der Lage ist, die Umwandlung von mindestens einem zweiten gelösten Stoff in den ersten gelösten Stoff zu fördern, um den osmotischen Druck in der Umhüllung zu verhindern; und
eine deformierbare Kammer (31) verbunden mit der Umhüllung, wobei die Kammer in der Lage ist, ihr Volumen unter der Wirkung des Lösungsmittels, welches sich von der Umhüllung in die Kammer bewegt,
durch Osmose zu vergrößern oder wobei die erwähnte Umhüllung ausgelegt ist, um mit dem Lösungsmittel in Kontakt zu sein, wobei die Kammer in der Lage ist, ihr Volumen unter der Wirkung des in die Umhüllung durch Osmose eindringenden Lösungsmittels zu vergrößern.

2. Stellglied nach Anspruch 1, wobei die Wand (14; 25) der Umhüllung (12; 61) für den zweiten gelösten Stoff permeabel ist.

3. Stellglied nach Anspruch 1, wobei die Wand (14; 25) der Umhüllung (12; 61) für den zweiten gelösten Stoff impermeabel ist, wobei der Katalysator in der Lage ist, die Transformation einer Anzahl von Teilchen des zweiten gelösten Stoffs in eine größere oder kleinere Anzahl von Teilchen des ersten gelösten Stoffs zu fördern.

4. Ein Motor (10, 40, 60), der das Stellglied des Anspruchs 1 aufweist, wobei die Kammer (31) Rückkehrmittel (32) aufweist, die der Volumenvergrößerung der Kammer entgegenwirken, und steuerbare Mittel (34) zur Absenkung des osmotischen Drucks in der Kammer.

5. Der Motor (40) nach Anspruch 4, wobei die erwähnte Wand (14) der Umhüllung (12) für den zweiten gelösten Stoff impermeabel ist, wobei der Katalysator in der Lage ist, die Transformation einer Anzahl von Teilchen des zweiten gelösten Stoffs in eine größere Anzahl von Teilchen des ersten gelösten Stoffs zu fördern, wobei der Motor ferner eine zusätzliche Umhüllung (42) aufweist und zwar mit einer Wand (44), die permeabel ist für das Lösungsmittel und impermeabel für die ersten und zweiten gelösten Stoffe und wobei die zusätzliche Umschließung oder Umhüllung ferner einen zusätzlichen Katalysator aufweist, der in der Lage ist, die Transformation einer Anzahl von Teilchen des ersten gelösten Stoffs in eine kleinere Anzahl von Teilchen des zweiten gelösten Stoffs zu fördern, wobei die zusätzliche Umhüllung mit der Kammer (61) durch ein Ventil (34) verbunden ist.

6. Der Motor (60) nach Anspruch 4, wobei die Wand (14) der Umhüllung (12) impermeabel für den zweiten gelösten Stoffl ist und wobei der Katalysator in der Lage ist, die Transformation einer Anzahl von Teilchen des zweiten gelösten Stoffs in eine größere Anzahl von Teilchen des ersten gelösten Stoffs zu fördern, und wobei die Umhüllung (12) in einer deformierbaren Umschließung (61) angeordnet ist, die das Lösungsmittel und den ersten gelösten Stoff enthält, wobei die Umschießung (61) einen zusätzlichen Katalysator enthält, der in der Lage ist, die Transformation einer Anzahl von Teilchen des ersten gelösten Stoffs in eine kleinere Anzahl von Teilchen des zweiten gelösten Stoffs zu fördern, wobei die Mittel (34) zur Absenkung des osmotischen Drucks in der Kammer ein Ventil (34) sind, welches in der Lage ist zur Verbindung der Kammer (31) und der Umhüllung.

7. Ein Motor (80), der das Stellglied gemäß Anspruch 1 aufweist, wobei die Umhüllung (61) mindestens teilweise deformierbar ist und verbunden ist mit der Kammer (31) auf dem Niveau der Wand (25), wobei der Motor erste Mittel (82, 84) aufweist zur Lieferung des Katalysators in die Umhüllung und zweite Mittel (86, 88) zur Lieferung eines zusätzlichen Katalysators, der in der Lage ist, die Transformation des ersten gelösten Stoffs in den zweiten gelösten Stoff in die Umhüllung zu fördern.

8. Der Motor nach Anspruch 7, wobei die Wand (25) für den zweiten gelösten Stoff permeabel ist, wobei der Katalysator in der Lage ist, die Transformation einer Anzahl von Teilchen des zweiten gelösten Stoffs in eine kleinere Anzahl von Teilchen des ersten gelösten Stoffs zu fördern, und wobei der zusätzliche Katalysator in der Lage ist, die Transformation einer Anzahl von Teilchen des ersten gelösten Stoffs in eine größere Anzahl von Teilchen des zweiten gelösten Stoffs zu fördern.

9. Der Motor nach Anspruch 7, wobei der zweite gelöste Stoff eine Verbindung ist, die eine Aminfunktion aufweist, wobei der erste gelöste Stoff ein Komplex des zweiten gelösten Stoffs und eines zusätzlichen Lösungsmittels ist und zwar eine Aldehyd-Funktion aufweisend, wobei ferner die Wand (25) impermeabel für das zusätzliche Lösungsmittel ist, und wobei der Katalysator das Wasserstoff-Ion ist, während der zusätzliche Katalysator das Hydroxyl-lon ist.

10. Der Motor nach Anspruch 9, wobei das erste Versorgungs- oder Liefermittel (82, 84) eine zusätzliche Umhüllung (84) aufweist, designiert oder konstruiert zur Aufnahme eines Glucose enthaltenden Lösungsmittels, wobei die zusätzliche Umhüllung Glucose-Oxidase-Enzyme enthält, die in der Lage sind, die Oxidation der Glucose zu fördern, um Gluconat-Ionen und Hydrogen-lonen vorzusehen.

11. Der Motor nach Anspruch 9, wobei die zweiten Versorgungsmittel oder Liefermittel (86, 88) eine zusätzliche Umhüllung (88) aufweisen, und zwar konstruiert zur Aufnahme eines Lösungsmittels welches Harnsäure enthält, wobei die zusätzliche Umhüllung Urease-Enzyme enthält, die in der Lage sind, die Oxidation von Harnsäure zu fördern, um AmmoniumIonen und Kohlenstoffdioxyd vorzusehen.
